# EUROPÄISCHE PATENTSCHRIFT

(11) **EP 1 812 099 B1**
(45) Veröffentlichungstag und Bekanntmachung des Hinweises auf die Patenterteilung: **23.01.2013**
(21) Anmeldenummer: 05809852.6
(22) Anmeldetag: 26.10.2005
(51) Int. Cl.: A61M 25/00, A61B 5/145

(54) **VENENVERWEILKATHETER**
INTRAVENOUS INDWELLING CATHETER
CATHETER VEINEUX A DEMEURE

(30) Priorität: 19.11.2004 DE 102004055989
(43) Veröffentlichungstag der Anmeldung: 01.08.2007
(73) Patentinhaber: Charité - Universitätsmedizin Berlin, 10117 Berlin (DE)
(72) Erfinder: NOTTER, Michael, 12205 Berlin (DE)
(74) Vertreter: Bittner, Thomas L.
(86) Internationale Anmeldenummer: PCT/DE2005/001945
(87) Internationale Veröffentlichungsnummer: WO 2006/053516

(56) Entgegenhaltungen:
- EP-B- 0 910 988
- US-A- 3 405 706
- US-A- 5 270 003
- US-A- 5 474 546
- US-A1- 2002 156 431
- US-B1- 6 296 624

## Beschreibung

Die Erfindung betrifft einen Verweilkatheter, insbesondere einen intravenösen Verweilkatheter für die Notfall-/Katastrophenversorgung.

### Hintergrund der Erfindung

Derartige Verweilkatheter umfassen üblicherweise ein Gehäuse mit einem in Längsrichtung des Gehäuses verlaufenden Gehäuseinnenraum, der sich zwischen einer vorderen und einer hinteren Öffnung des Gehäuses erstreckt und optional mit einem im Bereich der Gehäusewand gebildeten. Zuspritzventil verbunden ist, über die dem Patienten nach dem Setzen des Verweilkatheters Medikamente in flüssiger Form verabreicht werden können. In den Gehäuseinnenraum ist eine Kanüle zum Punktieren der Haut beim Setzen des Verweilkatheters eingeführt. Die Kanüle ist in den Gehäuseinnenraum soweit eingeführt, daß eine Eintrittsöffnung im Bereich einer Spitze der Kanüle außerhalb des Gehäuses und vor der vorderen Öffnung angeordnet ist. Ein hinteres Ende der Kanüle ist in einem Griffstück angeordnet, mit dem die Kanüle beim Einführen in das Gehäuse und beim Herausnehmen aus dem Gehäuse geführt werden kann. Das Griffstück ist üblicherweise in ein hinteres Ende des Gehäuses eingesteckt, wenn die Kanüle zum Setzen des Verweilkatheters in das Gehäuse eingeführt ist. In dem Griffstück ist ein Kanal gebildet, der mit der Austrittsöffnung am hinteren Ende der Kanüle in Verbindung steht.

In ein von dem Gehäuse abgewandten Ende des Griffstücks können verschiedene Abschluß - oder Anschlußstücke, beispielsweise ein Luer-Anschlußsystem, eingesteckt werden, um den Kanal in dem Griffstück zu verschließen und /oder andere Leitungen anzuschließen. Beim Setzen des Verweilkatheters ist in dem hinteren Ende des Griffstücks üblicherweise ein Abschußstück angeordnet, dessen Innenraum auf der einen Seite mit den Kanal in dem Griffstück und somit mit der Austrittsöffnung der Kanüle und auf der anderen Seite mit einer Ausströmöffnung verbunden ist. Im Bereich der Ausströmöffnung ist ein luftdurchlässiger Filter angeordnet, durch den Luft ausströmen kann, wenn aus dem Kanal in dem Griffstück Blut in das Abschlußstück fließt und hierdurch die Luft verdrängt. Nach dem Setzen des Verweilkatheters werden üblicherweise das Abschlußstück und das Griffstück mit der Kanüle entfernt, so daß an das Gehäuse anderer Anschlüsse angeschlossen werden können, um die weitere Patientenversorgung durchzuführen.

Verweilkatheter mit einem solchen Aufbau sind in verschiedenen Ausführungsformen bekannt. Üblicherweise ist an dem Gehäuse des Verweilkatheters darüber hinaus eine Stützauflage, beispielsweise in Form von seitlich abstehenden Flügeln, gebildet, um eine Auflage des Verweilkatheters auf der Haut des Patienten zu stützen.

Verweilkatheter werden sowohl im Bereich der stationären Patientenversorgung als auch in Verbindung mit der Versorgung von Patienten im Notfall- oder Katastropheneinsatz genutzt. Bei der Versorgung schwerverletzter Patienten entsteht häufig unmittelbar nach Aufnahme im stationären Behandlungszentrum die Indikation für Notfalltransfusionen, vor allem mit Erythrozytenkonzentraten. Diese können sich dann zu Massivtransfusionen ausweiten. Hierbei ist es notwendig, schnellstmöglich mit AB0-kompatiblen Konserven transfundieren zu können. Dieser Standard setzt die Bestimmung der Notfallblutgruppe (Antigenbestimmung auf Empfängererythrozyten) voraus. Hierfür werden lediglich 0,1 - 0,5 ml natürliches Blut des Patienten benötigt. Die Praxis zeigt, daß die notfalltransfusionsmedizinische Versorgung durch folgende Umstände erschwert ist:
- Die Infusion mit dem Plasmaexpander Hydroxyäthylstärke (HES), der zur Schockbehandlung am Unfallort eingesetzt wird, erschwert die zweifelsfreie Blutgruppenbestimmung oder macht sie in kurzer Zeit sogar unmöglich, weil HES-Moleküle mit hohem Molekulargewicht Erythrozyten agglutinieren können (Pseudoagglutination).
- Patienten in schwerem hämorrhagischen Schock erhalten unmittelbar nach Aufnahme im Rettungszentrum sogenannte "0 Rh negativ Notfallkonserven". Nach der Transfusion von Notfallkonserven kann die Blutgruppe des Patienten nicht mehr zweifelsfrei bestimmt werden (Mischfeldagglutination). Die weitere Versorgung kann dann nicht mehr mit AB0-kompatiblen, sondern muß mit Konserven der Blutgruppe 0 erfolgen. Insbesondere die gleichzeitige Versorgung mehrerer Patienten oder das Transfusionsmanagement im Katastrophenfall oder die gleichzeitige Versorgung mehrerer Patienten wird dadurch erschwert.
- Idealerweise würde die Prätransfusionsblutprobe für die Notfallblutgruppenbestimmung am mobilen Einsatzort im Rahmen der Primärversorgung abgenommen werden. Die Praxis der Rettungsmedizin zeigt jedoch, daß dies nicht geschieht, weil die Sicherung der Vitalfunktionen des Patienten Vorrang hat. Das Befüllen, verwechslungssicheres Beschriften und Aufbewahren von Blutröhrchen stellt in der Notfallprimärversorgung eine praktisch nicht erfüllbare Aufgabe dar. Deshalb erfolgt bei den meisten Patienten die Blutabnahme erst bei Einlieferung des Patienten im stationären Behandlungszentrum.

Von dem Risiko der nicht oder nicht eindeutig bestimmbaren Blutgruppe im Notfall sind am ehesten Patienten mit schwerem hämorrhagischen Schock betroffen. Sie benötigen Plasmaexpander in großer Menge und Notfallkonserven am dringendsten.

Das Dokument US 3,405,706 A beschreibt eine Blutentnahmevorrichtung mit einem Gehäuse und einer Nadel zum Punktieren einer Haut eines Patienten. In dem Gehäuse ist ein mit der Nadel in Verbindung stehender Kanal angeordnet. Über die Länge des Kanals angeordnet gehen mehrere Hilfsnadeln senkrecht von dem Kanal ab. Beabstandet von einer jeweiligen dem Kanal abgewandeten Spitze der Hilfsnadeln sind Entnahmebauteile angeordnet, wobei die Entnahmebauteile jeweils mit einem Verschluss vakuumversiegelt sind.

Aus dem Dokument US 6,296,624 B1 ist eine Vorrichtung zur Entnahme von Flüssigkeitsproben mit einem Gehäuse und einer in dem Gehäuse angeordneten Nadel, wobei an der Nadel ein lösbar befestigter Behälter zum Aufnehmen der Flüssigkeit angeordnet ist.

Aufgabe der Erfindung ist es, ein verbessertes Verweilkatheters zu schaffen, mit dem eine zuverlässige Entnahme einer Blutprobe gewährleistet wird, insbesondere auch in Notfall- oder Katastropheneinsätzen.

Diese Aufgabe wird erfindungsgemäß durch ein Vcrweilkatheter nach dem unabhängigen Anspruch 1 gelöst.

Die Erfindung umfaßt den Gedanken eines Verweilkatheters, insbesondere eines intravenösen Verweilkatheters zur Notfallversorgung, mit einem Gehäuse und einer Kanüle zum Punktieren, die herausnehmbar in einen Gehäuseinnenraum in Längsrichtung des Gehäuses eingeführt ist und sich durch eine vordere Gehäuseöffnung erstreckt, so daß eine Eintrittsöffnung der Kanüle zumindest in einer vollständig eingeführten Stellung der Kanüle außerhalb des Gehäuses angeordnet ist, wobei ein Entnahmebauteil zum Speichern einer Blutprobe vorgesehen ist, welches an dem Gehäuse mit Hilfe einer flexiblen Befestigung bewegbar gehalten wird und welches zwischen einer gekoppelten Stellung, in der ein in den Entnahmebauteil gebildetes, selbstfüllendes Reservoir zum Aufnehmen einer Probe eines die Kanüle durchströmenden Blutvolumens mit einer Austrittsöffnung der Kanüle in Verbindung steht, und einer entkoppelten Stellung verlagerbar ist, in der das Reservoir von der Austrittsöffnung der Kanüle getrennt ist und das Entnahmebauteil mittels der flexiblen Befestigung an dem Gehäuse gehalten wird.

In der gekoppelten Stellung ist das Entnahmebauteil an ein Griffstück gekoppelt, mit dem die Kanüle beim Einführen in das / Herausnehmen aus dem Gehäuse geführt werden kann. In der entkoppelten Stellung ist das Entnahmebauteil von dem Griffstück getrennt.

Ein wesentliche Vorteil, welcher mit Hilfe der Erfindung gegenüber dem Stand der Technik erreicht wird, besteht darin, daß mit dem vorgesehenen Entnahmebauteil eine Blutprobe des Patienten beim Setzen des Verweilkatheters entnommen und in dem selbstfüllenden Reservoir gespeichert wird, wobei das hierbei entnommene Blut noch nicht mit später im Rahmen der Patientenversorgung zu verabreichenden Stoffen vermischt ist. Die auf diese Weise in dem Reservoir des Entnahmebauteils gespeicherte Blutprobe wird mit Hilfe der Befestigung an dem Gehäuse des gesetzten Verweilkatheters und somit an dem Patienten gehalten. Hierdurch kann sichergestellt werden, daß für eine spätere Blutuntersuchung, insbesondere der Fcststellung der Blutgruppe des Patienten, eine Prätransfusionsblutprobe vor jeder Verabreichung von Infusionslösung oder von Notfallkonserven zur Verfügung gestellt wird.

Da das Entnahmebauteil mit dem Reservoir, in dem die Blutprobe gespeichert ist, an den gesetzten Verweilkatheter befestigt ist, wird ein Verlust der Blutprobe vermieden. Auch die Zuordnung der später untersuchten Blutprobe zu dem Patienten wird auf diese Weise sichergestellt. Erst wenn die Blutuntersuchung durchgeführt werden soll, wird das Entnahmebauteil von dem gesetzten Verweilkatheter gelöst. Bis zu diesem Zeitpunkt wird ein Verlust der Blutprobe aufgrund der Befestigung des Entnahmebauteils an dem Gehäuse des Verweilkatheters unterbunden.

Die beschriebenen Vorteile entfalten ihre Wirkung insbesondere in Verbindung mit dem Einsatz des Verweilkatheters bei Notfall- oder Katastropheneinsätzen, bei denen zunächst die schnelle Notversorgung der Patienten im Vordergrund steht und eine Blutuntersuchung erst später im Krankenhaus durchgeführt wird.

Eine zweckmäßige Ausgestaltung der Erfindung kann vorsehen, daß die Befestigung eine Sollbruchstelle aufweist, wodurch das Lösen des Entnahmebauteils mit der im Reservoir gespeicherten Blutprobe von dem Gehäuse des Verweilkatheters erleichtert wird. Das Entnahmebauteil kann von dem Gehäuse ohne weitere Hilfsmittel, beispielsweise eine Schere, getrennt werden.

Um hohen hygienischen und medizinischen Ansprüchen zu entsprechen, sieht eine Ausgestaltung der Erfindung vor, daß die Befestigung aus einem Kunststoffmaterial ist. Beispielsweise kann es sich um ein Kunststoffband handeln. Dieses hat darüber hinaus den Vorteil, daß die Befestigung aus dem gleichen Material ist, aus dem üblicherweise das Gehäuse und die meisten anderen Teile des Verweilkatheters hergestellt wird.

Eine zweckmäßige Weiterbildung der Erfindung sieht vor, daß an dem Gehäuse ein Haltebauteil zum Aufnehmen des Entnahmebauteils gebildet ist, um das Entnahmebauteil entkoppelt von der Austrittsöffnung der Kanüle an dem Gehäuse zu lagern. Auf diese Weise wird verhindert, daß das Entnahmebauteil nach dem Setzen des Verweilkatheters und dem Entfernen der Kanüle an dem Gehäuse lose herumhängt. Mit Hilfe des Haltebauteils wird das Entnahmebauteil örtlich fixiert.

Ein mit wenig Aufwand durchführbares Anordnen des Entnahmebauteils in dem Haltebauteil / Herausnehmen des Entnahmebauteils aus dem Haltebauteil wird bei einer bevorzugten Ausführungsform der Erfindung dadurch erreicht, daß das Haltebauteil eine Steckaufnahme für das Entnahmebauteil aufweist, beispielsweise einen Luer-Anschluß. Zwischen Haltebauteil und Entnahmebauteil wird somit eine Steckverbindung gebildet, indem das Entnahmebauteil in das Haltebauteil zumindest teilweise eingesteckt wird.

Zweckmäßig sieht eine Fortbildung der Erfindung vor, daß das Haltebauteil auf einer an dem Gehäuse angeordneten Stützauflage gebildet ist. Hierdurch ist das Haltebauteil in einem Bereich angeordnet, welcher zur Auflage des Verweilkatheters auf der Haut des Patienten dient, so daß beim Vorsehen des Entnahmebauteils eine Änderung des übrigen konstruktiven Aufbaus des Gehäuses des Verweilkatheters nicht notwendig ist.

Ein teilweiser Verlust der entnommenen Blutprobe und / oder eine mögliche Verschmutzung der Blutprobe wird bei einer vorteilhaften Ausgestaltung der Erfindung dadurch vermieden, daß das Haltebauteil als Blindstopfen für das Entnahmebauteil gebildet ist. Auf diese Weise wird das Eindringen von Verschmutzungen in das Reservoir mit der Blutprobe verhindert.

Eine bevorzugte Ausführungsform der Erfindung kann vorsehen, daß an dem Entnahmebauteil eine Markierungsfläche gebildet ist. Im Bereich der Markierungsfläche können Informationen über den Patienten aufgeschrieben werden, beispielsweise mit Hilfe eines wasserfesten Stiftes, so daß bei der späteren Blutuntersuchung eine Verwechslung der Blutproben vermieden ist. Im Behandlungszentrum kann auf die Markierungsfläche die zentrumseigene Patienteninformation (Klebeetikett) aufgebracht werden.

Bei einer zweckmäßigen Ausführungsform der Erfindung ist vorgesehen, daß das Entnahmebauteil als Abschlußstück zum Verschließen eines mit der Austrittsöffnung der Kanüle in Verbindung stehenden Hohlraums gebildet ist, wobei der Hohlraum mit dem Reservoir in Verbindung steht, wenn das Entnahmebauteil in der gekoppelten Stellung angeordnet ist. Hierdurch übernimmt das Entnahmebauteil nicht nur die Funktion zur Speicherung der Blutprobe sondern auch die eines Abschluß- / Endstücks beim Setzen des Verweilkatheters.

Ein hinsichtlich seiner Funktionalitäten erweitertes Entnahmebauteil ist bei einer Ausgestaltung der Erfindung dadurch gebildet, daß im Bereich einer Ausströmöffnung des Abschluß-Ein hinsichtlich seiner Funktionalitäten erweitertes Entnahmebauteil ist bei einer Ausgestaltung der Erfindung dadurch gebildet, daß im Bereich einer Ausströmöffnung des Abschlußstücks ein luftdurchlässiger Filter angeordnet ist, durch den Luft beim Einströmen des Blutvolumens aus der Kanüle in den Hohlraum entweichen kann.

Die Entnahme der in dem Reservoir des Entnahmebauteils gespeicherten Blutprobe zur Durchführung der Untersuchung ist bei einer bevorzugten Weiterbildung dadurch erleichtert, daß das Entnahmeteil eine mit dem Reservoir in Verbindung stehende, verschließbare Entnahmeöffnung zum Entnehmen der Blutprobe aufweist.

Eine Fortbildung der Erfindung kann zweckmäßig darin bestehen, daß in dem Reservoir eine Menge von EDTA-Kristallen als Antikoagulans angeordnet ist, um für die Blutgruppenbestimmung antikoaguliertes Blut zur Verfügung zu haben.

### Beschreibung von Ausführunssbeispielen

Die Erfindung wird im folgenden anhand von Ausführungsbeispielen unter Bezugnahme auf die Figuren der Zeichnung näher erläutert. Hierbei zeigen:
Fig. 1 eine schematische Darstellung eines Verweilkatheters vor dem Punktieren; und
Fig. 2 eine schematische Darstellung des Verweilkatheters nach Fig. 1 nach dem Punktieren.

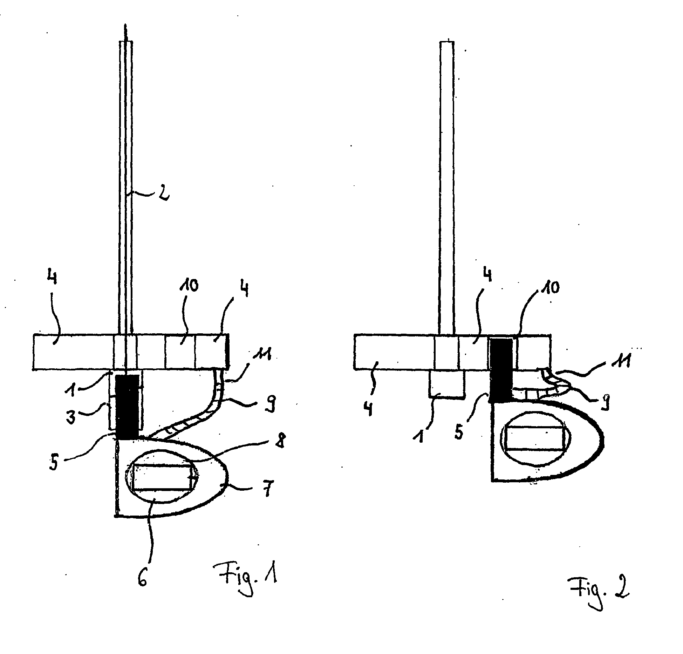

Fig.1 zeigt eine schematische Darstellung eines Verweilkatheters mit einem Gehäuse 1, in welches eine Kanüle 2 mit Hilfe eines Griffstücks 3 eingeführt ist. Das Griffstück 3 dient zur Handhabung der Kanüle 2 beim Einführen in das Gehäuse 1 sowie beim Herausnehmen aus dem Gehäuse 1. An dem Gehäuse 1 sind Seitenflügel 4 gebildet, die zur Unterstützung der Auflage des Verweilkatheters beim Setzen des Verweilkatheters und danach auf der Haut des Patienten dienen. Das Gehäuse 1 des Verweilkatheters, einschließlich der Seitenflügel 4 ist üblicherweise als ein Bauteil aus einem Kunststoffmaterial hergestellt. 5 gebildetes Reservoir 7 mit einer Austrittsöffnung am hinteren Ende der Kanüle 2 in Verbindung steht, wodurch ein die Kanüle 2 durchströmendes Blutvolumen teilweise in das Reservoir 7 gelangen kann und dort als Blutprobe gespeichert wird. Diese Blutprobe wird unmittelbar beim Setzen des Verweilkatheters entnommen, so daß es sich um eine Prätransfusionsblutprobe handelt. Das Reservoir 7 füllt sich beim Setzen des Verweilkatheters selbsttätig.

An dem Entnahmebauteil 5 ist eine Markierungsfläche 6 gebildet, die dazu dient, Patienteninformationen aufzuschreiben und / oder den Namen der behandelnden Person (Arzt, Sanitäter) oder eine Etikette mit den patientenidentifizierenden Informationen aufzukleben. Im Bereich der Markierungsfläche 6 ist darüber hinaus ein Deckel 8 vorgesehen, der geöffnet werden kann, um die in dem Reservoir 7 gespeicherte Blutprobe für die Blutuntersuchung im Labor zu entnehmen.

Das Entnahmebauteil 5 ist über eine flexible Befestigung 9 mit den rechten Seitenflügel 4 verbunden. Bei der flexiblen Befestigung 9 handelt es sich beispielsweise um eine Plastiklasche oder ein Plastikband. Die flexible Befestigung 9 stellt sicher, daß das Entnahmebauteil 5 nach dem Herausnehmen aus dem Griffstück 3 weiter an dem Gehäuse 1 des Verweilkatheters gehalten wird und so nicht verloren gehen kann. Gemäß Fig. 2 ist auf einem der Seitenflügeln 4 ein Haltebauteil 10 gebildet, in welches das Entnahmebauteil 5 eingesteckt ist, um dessen örtlich fixierte Lagerung an dem Gehäuse 1 sicher zu stellen. Hierbei ist das Haltebauteil 10 vorzugsweise als Blindstopfen ausgeführt, um ein Herauslaufen von Blut aus dem Entnahmebauteil 5 zu verhindern.

Die flexible Verbindung 9 verfügt über eine Sollbruchstelle 11, so daß das Entnahmebauteil 5 von dem Gehäuse 1 getrennt werden kann, wenn die Blutuntersuchung vorbereitet werden soll. Dieses geschieht beispielsweise nach dem Eintreffen des Patienten in einem Krankenhaus.

Die Größe des Reservoirs 7 muß lediglich ausreichend sein, um wenige Milliliter Blut aufzunehmen, da bereits eine solche Menge für eine Blutgruppenbestimmung ausreichend ist. Das Reservoir 7 enthält auch eine definierte Menge von EDTA-Kristallen, um die Antikogenladien der Blutprobe zu bewirken.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Verweilkatheter, insbesondere intravenöser Verweilkatheter zur Notfallversorgung, mit:
- einem Gehäuse (1),
- einer Kanüle (2) zum Punktieren, die in einem Griffstück (3) aufgenommen ist, mit dem die Kanüle (2) beim Einführen in das oder beim Herausnehmen aus dem Gehäuse (1) geführt werden kann, und
- einem Entnahmebauteil (5) zum Speichern einer Blutprobe, welches zwischen einer gekoppelten und einer entkoppelten Stellung verlagerbar ist, wobei:
- in der gekoppelten Stellung das Entnahmebauteil (5) an das Griffstück (3) gekoppelt ist und ein in dem Entnahmebauteil (5) gebildetes, selbstfüllendes Reservoir (7) zum Aufnehmen einer Probe eines die Kanüle (2) durchströmenden Blutvolumens mit einer Austrittsöffnung der Kanüle (2) in Verbindung steht und
- in der entkoppelten Stellung das Reservoir (7) von der Austrittsöffnung der Kanüle (2) und von dem Griffstück (3) getrennt ist,
**dadurch gekennzeichnet, daß**
- die Kanüle (2) herausnehmbar in einen Gehäuseinnenraum in Längsrichtung des Gehäuses (1) eingeführt ist und sich durch eine vordere Gehäuseöffnung erstreckt, so daß eine Eintrittsöffnung der Kanüle (2) zumindest in einer vollständig eingeführten Stellung der Kanüle (2) außerhalb des Gehäuses (1) angeordnet ist,
- eine flexible Befestigung (9) gebildet ist, mit der das Entnahmebauteil (5) an dem Gehäuse (1) bewegbar zwischen der gekoppelten und der entkoppelten Stellung gehalten wird, und
- das Entnahmebauteil (5) in der entkoppelten Stellung mittels der flexiblen Befestigung (9) an dem Gehäuse (1) gehalten wird.

2. Verweilkatheter nach Anspruch 1, **dadurch gekennzeichnet, daß** die flexible Befestigung (9) eine Sollbruchstelle (11) aufweist.

3. Verweilkatheter nach Anspruch 1 oder 2, **dadurch gekennzeichnet, daß** die flexible Befestigung (9) aus einem Kunststoffmaterial ist.

4. Verweilkatheter nach einem der vorangehenden Ansprüche, **dadurch gekennzeichnet, daß** an dem Gehäuse (1) ein Haltebauteil (10) zum Aufnehmen des Entnahmebauteils (5) gebildet ist, um das Entnahmebauteil (5) entkoppelt von der Austrittsöffnung der Kanüle (2) an dem Gehäuse (1) zu lagern.

5. Verweilkatheter nach Anspruch 4, **dadurch gekennzeichnet, daß** das Haltebauteil (10) eine Steckaufnahme für das Entnahmebauteil (5) aufweist.

6. Verweilkatheter nach Anspruch 4 oder 5, **dadurch gekennzeichnet, daß** das Haltebauteil (10) auf einer an dem Gehäuse (1) angeordneten Stützauflage (4) gebildet ist.

7. Verweilkatheter nach einem der Ansprüche 4 bis 6, **dadurch gekennzeichnet, daß** das Haltebauteil (10) als Blindstopfen für das Entnahmebauteil (5) gebildet ist.

8. Verweilkatheter nach einem der vorangehenden Ansprüche, **dadurch gekennzeichnet, daß** an dem Entnahmebauteil (5) eine Markierungsfläche (6) gebildet ist.

9. Verweilkatheter nach einem der vorangehenden Ansprüche, **dadurch gekennzeichnet, daß** das Entnahmebauteil (5) als Abschlußstück zum Verschließen eines mit der Ausströmöffnung der Kanüle (2) in Verbindung stehenden Hohlraums gebildet ist, wobei der Hohlraum mit dem Reservoir (7) in Verbindung steht, wenn das Entnahmebauteil (5) in der gekoppelten Stellung angeordnet ist.

10. Verweilkatheter nach Anspruch 9, **dadurch gekennzeichnet, daß** im Bereich einer Ausströmöffnung des Abschlußtücks ein luftdurchlässiger Filter angeordnet ist, durch den Luft beim Einströmen des Blutvolumens aus der Kanüle (2) in den Hohlraum entweichen kann.

11. Verweilkatheter nach einem der vorangehenden Ansprüche, **dadurch gekennzeichnet, daß** das Entnahmeteil (5) eine mit dem Reservoir (7) in Verbindung stehende, verschließbare Entnahmeöffnung (8) zum Entnehmen der Blutprobe aufweist.

12. Verweilkatheter nach einem der vorangehenden Ansprüche, **dadurch gekennzeichnet, daß** in dem Reservoir (7) eine Menge von EDTA-Kristallen als Antikoagulans angeordnet ist.

## Claims

1. An indwelling catheter, in particular an intravenous indwelling catheter for emergency treatment, comprising:
- a housing (1),
- a puncturing cannula (2), wherein the cannula (2) is received in a grip piece (3) which serves for handling of the cannula (2) as it is introduced into the housing (1) and as it is removed from the housing (1), and
- a collection component (5) for storing a blood sample which is displaceable between a coupled position and an uncoupled position, wherein:
- in the coupled position the collection component (5) is coupled to the grip piece (3) und a self-filling reservoir (7) formed in the collection component (5) is connected to an exit opening of the cannula (2) in order to receive a sample of a volume of blood flowing through the cannula (2) and
- in the uncoupled position the reservoir (7) is separated from the exit opening of the cannula (2) and the grip piece (3),
**characterized in that**
- the cannula (2) is removably introduced into a housing interior in the longitudinal direction of the housing (1) and extends through a front housing opening in such a way that an entry opening of the cannula (2) is arranged outside the housing (1) at least in a fully introduced position of the cannula (2),
- a flexible fixing element (9) is formed by which the collection component (5) is held in on the housing (1) being movable between the coupled position and the uncoupled position, and
- the collection component (5) is hold in the uncoupled position on the housing (1) by means of the flexible fixing element (9).

2. The indwelling catheter according to claim 1, **characterized in that** the flexible fixing element (9) has a predetermined breaking point (11).

3. The indwelling catheter according to claim 1 or 2, **characterized in that** the flexible fixing element (9) is made of a plastic material.

4. The indwelling catheter according to any one of the preceding claims, **characterized in that** a holding component (10) for holding the collection component (5) is formed on the housing (1), in order to store the collection component (5) on the housing (1) in a manner uncoupled from the exit opening of the cannula (2).

5. The indwelling catheter according to claim 4, **characterized in that** the holding component (10) has an insertion opening for the collection component (5).

6. The indwelling catheter according to claim 4 or 5, **characterized in that** the holding component (10) is formed on a support (4) arranged on the housing (1).

7. The indwelling catheter according to any one of claims 4 to 6, **characterized in that** the holding component (10) is formed as a blind stopper for the collection component (5).

8. The indwelling catheter according to any one of the preceding claims, **characterized in that** a marking area (6) is formed on the collection component (5).

9. The indwelling catheter according to any one of the preceding claims, **characterized in that** the collection component (5) is formed as a closure piece for closing a hollow chamber which is connected to the outflow opening of the cannula (2), wherein the hollow chamber is connected to the reservoir (7) when the collection component (5) is in the coupled position.

10. The indwelling catheter according to claim 9, **characterized in that** an air-permeable filter is arranged in the region of an outflow opening of the closure piece, through which filter air can escape out of the cannula (2) and into the hollow chamber during the inflow of the volume of blood.

11. The indwelling catheter according to any one of the preceding claims, **characterized in that** the collection component (5) has a closable removal opening (8) connected to the reservoir (7) in order to remove the blood sample.

12. The indwelling catheter according to any one of the preceding claims, **characterized in that** a quantity of EDTA crystals is arranged in the reservoir (7) as an anticoagulant.

## Revendications

1. Cathéter permanent, en particulier cathéter permanent intraveineux pour les soins d'urgence, comprenant :
- un logement (1),
- une canule (2) pour la ponction qui est reçue dans un composant de poignée (3) avec laquelle la canule (2) peut être dirigée lors de l'insertion dans le logement (1) ou lors de l'enlèvementhors de celui-ci, et
- un composant de prélèvement (5) pour stocker un échantillon sanguin, lequel peut être déplacé entre une position couplée et une position découplée, sachant que :
- dans la position couplée, le composant de prélèvement (5) est coupléau composant de poignée (3),et un réservoir (7) à auto-remplissage formé dans le composant de prélèvement (5) pour recevoir un échantillon d'un volume sanguin circulant à travers la canule (2) est en liaison avec une ouverture de sortie de la canule (2) et
- dans la position découplée, le réservoir (7) est séparé de l'ouverture de sortie de la canule (2) et du composant de poignée (3),
**caractérisé en ce que**
- la canule (2) est introduite de manière à pouvoir être sortie dans un espace intérieur du logement, dans le sens longitudinal du logement (1) et s'étend à travers une ouverture de logement avant, de façon à ce que l'ouverture d'entrée de la canule (2) soit disposée hors du logement (1) au moins dans uneposition complètement introduite de la canule (2),
- une fixation flexible (9) est formée, avec laquelle le composant de prélèvement (5) est maintenuen pouvant se déplacer entre la position couplée et la position découplée, et
- le composant de prélèvement (5) dans la position découplée est maintenu sur le logement (1) via la fixation flexible (9).

2. Cathéter permanent selon la revendication 1, **caractérisé en ce que** la fixation flexible (9) présente un point de rupture nominal (11).

3. Cathéter permanent selon la revendication 1 ou 2, **caractérisé en ce que** la fixation flexible (9) est fabriquée dans un matériau plastique.

4. Cathéter permanent selon l'une des revendications précédentes, **caractérisé en ce qu'**un composant de fixation (10) pour recevoir le composant de prélèvement (5) est formé sur le logement (1), pour conserver sur le logement (1) le composant de prélèvement (5) découplé de l'ouverture de sortie de la canule (2).

5. Cathéter permanent selon la revendication 4, **caractérisé en ce que** le composant de fixation (10) présente une réception à enfichage pour le composant de prélèvement (5).

6. Cathéter permanent selon la revendication 4 ou 5, **caractérisé en ce que** le composant de fixation (10) est formé sur un appui de soutien (4) disposé sur le logement (1).

7. Cathéter permanent selon l'une des revendication 4 à 6, **caractérisé en ce que** le composant de fixation (10) est formé en tant que bouchon borgnepour le composant de prélèvement (5).

8. Cathéter permanent selon l'une des revendications précédentes, **caractérisé en ce qu'**une surface de marquage (6) est formée sur le composant de prélèvement (5).

9. Cathéter permanent selon l'une des revendications précédentes, **caractérisé en ce que** le composant de prélèvement (5) est formé en tant que pièce de fermeture pour fermer un espace creux en liaison avec l'ouverture d'écoulement de sortie de la canule (2), sachant que l'espace creux est en liaison avec le réservoir (7) lorsque le composant de prélèvement (5) est disposé dans la position couplée.

10. Cathéter permanent selon la revendication 9, **caractérisé en ce qu'**un filtre perméable à l'air est disposé au niveaud'une ouverture d'écoulement de sortie du composant de fermeture, à travers lequel de l'air peut s'échapper de la canule (2) dans l'espace creux lors de l'entrée du volume sanguin.

11. Cathéter permanent selon l'une des revendications précédentes, **caractérisé en ce que** le composant de prélèvement (5) présente une ouverture de prélèvement (8) en liaison avec le réservoir (7) et pouvant être fermée, pour prélever l'échantillon sanguin.

12. Cathéter permanent selon l'une des revendications précédentes, **caractérisé en ce qu'**une quantité de cristaux EDTA en tant qu'anticoagulants est disposée dans le réservoir (7).
